# EUROPÄISCHE PATENTSCHRIFT

(11) **EP 0 578 137 B1**
(45) Veröffentlichungstag und Bekanntmachung des Hinweises auf die Patenterteilung: **16.10.1996**
(21) Anmeldenummer: 93110549.8
(22) Anmeldetag: 01.07.1993
(51) Int. Cl.: C12P 17/12, C12P 7/40

(54) **Mikrobiologisches Verfahren zur Herstellung von 5-Hydroxy-2-pyrazincarbonsäure**
Microbiological process for the production of 5-hydroxy-2-pyrazine carboxylic acid
Procédé microbiologique de la production d'acide carboxylique de 5-hydroxy-2-pyrazine

(30) Priorität: 08.07.1992 CH 2150/92
(43) Veröffentlichungstag der Anmeldung: 12.01.1994
(73) Patentinhaber: LONZA AG, CH-3945 Gampel/Wallis (CH)
(72) Erfinder: Kiener, Andreas, Dr., Visp, (Kanton Wallis) (CH)
(74) Vertreter: Weinhold, Peter, Dr.

(56) Entgegenhaltungen:
- EP-A- 0 519 512
- EP-A- 0 529 653
- PATENT ABSTRACTS OF JAPAN, unexamined applications, C Field, Band 12, Nr. 148, 7. May 1988 THE PATENT OFFICE JAPANESE GOVERNMENT Seite 36 C 493

## Beschreibung

Die Erfindung betrifft ein neues Verfahren zur Herstellung von 5-Hydroxypyrazin-2-carbonsäure und/oder deren Salze mit 3-Cyanpyridin verwertenden Mikroorganismen ausgehend von 2-Cyanpyrazin.

Im folgenden wird unter 5-Hydroxypyrazin-2-carbonsäure auch deren Salze wie beispielsweise deren Alkalisalze oder Ammoniumsalze verstanden.

5-Hydroxypyrazin-2-carbonsäure kann beispielsweise als Zwischenprodukt zur Herstellung von Pharmazeutika, wie beispielsweise zur Herstellung von Pyrazin-Nukleosidanalogen mit cytostatischer Wirkung (M. Bobek, J. Heterocyclic Chem., 1991, 28, S. 1131), verwendet werden.

Bekannt ist, dass 5-Hydroxypyrazin-2-carbonsäure im Metabolismus von Hunden und Menschen aus Pyrazinamid über Pyrazincarbonsäure gebildet wird (J.Pharmacol. Exp. Ther., 1972, 180(2), 411-434).

Ein mikrobiologisches Verfahren zur Herstellung von 5-Hydroypyrazin-2-carbonsäure ist aus der EP-A 0 519 512 bekannt. Dieses Dokument fällt unter Art. 54(3) EPÜ und ist für die Frage der erfinderischen Tätigkeit nicht von Bedeutung.

Bei diesem Verfahren wird 5-Hydroxypyrazin-2-carbonsäure ausgehend von Pyrazincarbonsäure mittels Nikotinsäure verwertenden Mikroorganismen hergestellt.

Ein grosser Nachteil dieses Verfahrens besteht darin, dass das Edukt, die Pyrazincarbonsäure, chemisch schwer zugänglich ist.

Aufgabe der vorliegenden Erfindung war, sowohl ein einfaches und wirtschaftliches als auch ein ökologisches Verfahren zur Herstellung von 5-Hydroxypyrazin-2-carbonsäure zur Verfügung zu stellen.

Diese Aufgabe wurde mit einem neuen Verfahren gemäss Patentanspruch 1 gelöst.

Erfindungsgemäss wird vorgeschlagen, 2-Cyanpyrazin als Substrat mit Mikroorganismen, die mit 3-Cyanpyridin als einzige Kohlenstoff-, Stickstoff- und Energiequelle wachsen, in 5-Hydroxypyrazin-2-carbonsäure zu überführen, wobei letzteres im Medium akkumuliert wird.

Prinzipiell sind für das Verfahren alle Mikroorganismen geeignet, die befähigt sind mit 3-Cyanpyridin als einzige Kohlenstoff-, Stickstoff- und Energiequelle zu wachsen und dieses über das entsprechende 6-Hydroxypyridincarbonsäure-Derivat abbauen.
Der Begriff "Mikroorganismen, die mit 3-Cyanpyridin als einzige Kohlenstoff-, Stickstoff- und Energiequelle wachsen", umfasst sowohl Mischungen von Mikroorganismen als auch Rein- Isolate dieser Mikroorganismen, die unter sterilen oder unsterilen Fermentationsbedingungen eingesetzt werden können.

Zweckmässig wird die Umsetzung mit Mikroorganismen der Spezies Agrobacterium (Agrobacterium sp.) DSM 6336 oder deren Deszendeten und Mutanten durchgeführt. Diese wurden bei der Deutschen Sammlung für Mikroorganismen und Zellkulturen GmbH (DSM), Marscheroderweg 1b, D-3300 Braunschweig am 07.02.1991 mit der Bezeichnung DSM 6336 hinterlegt.

### Wissenschaftliche Beschreibung von Agrobacterium sp. (DSM 6336)

Eigenschaften des Stammes:

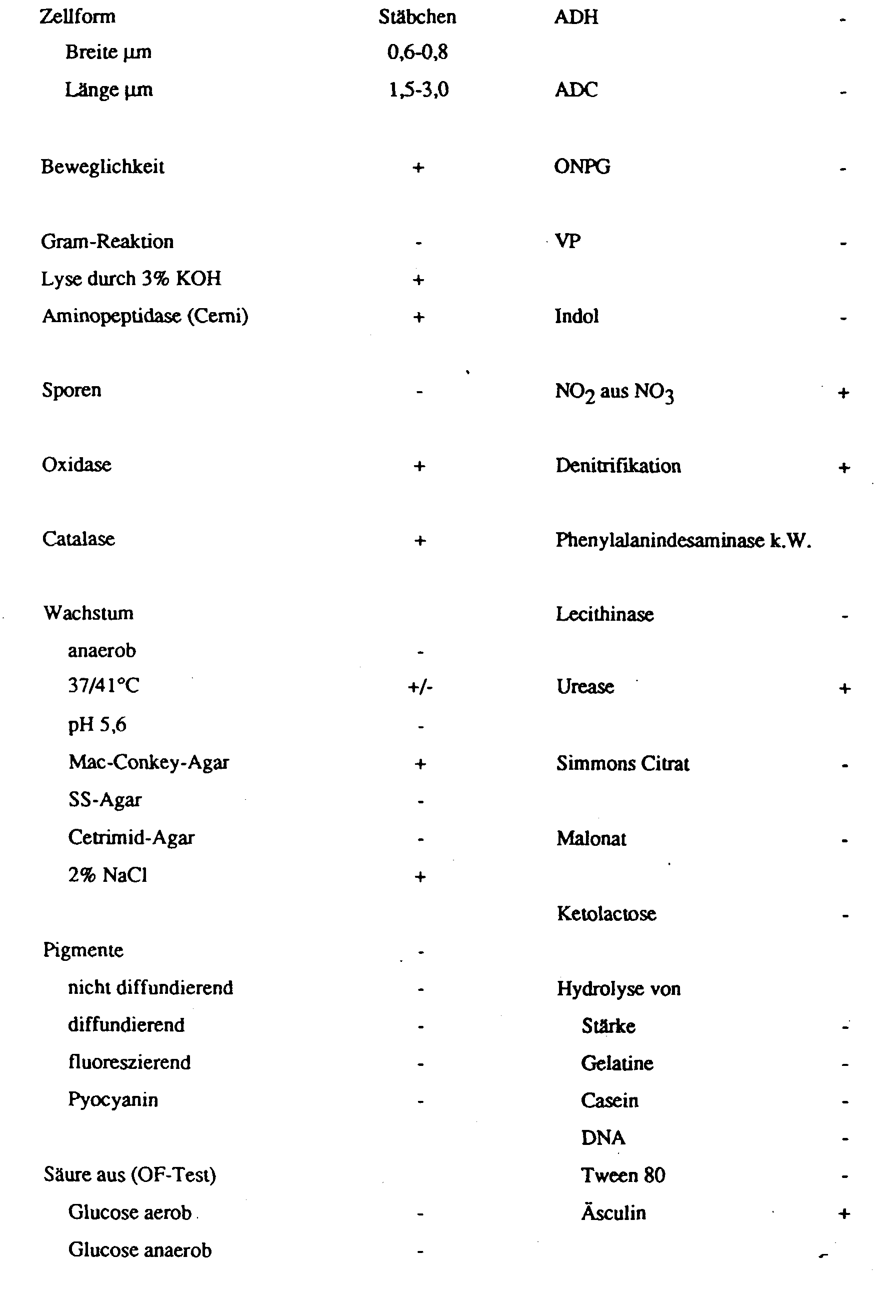

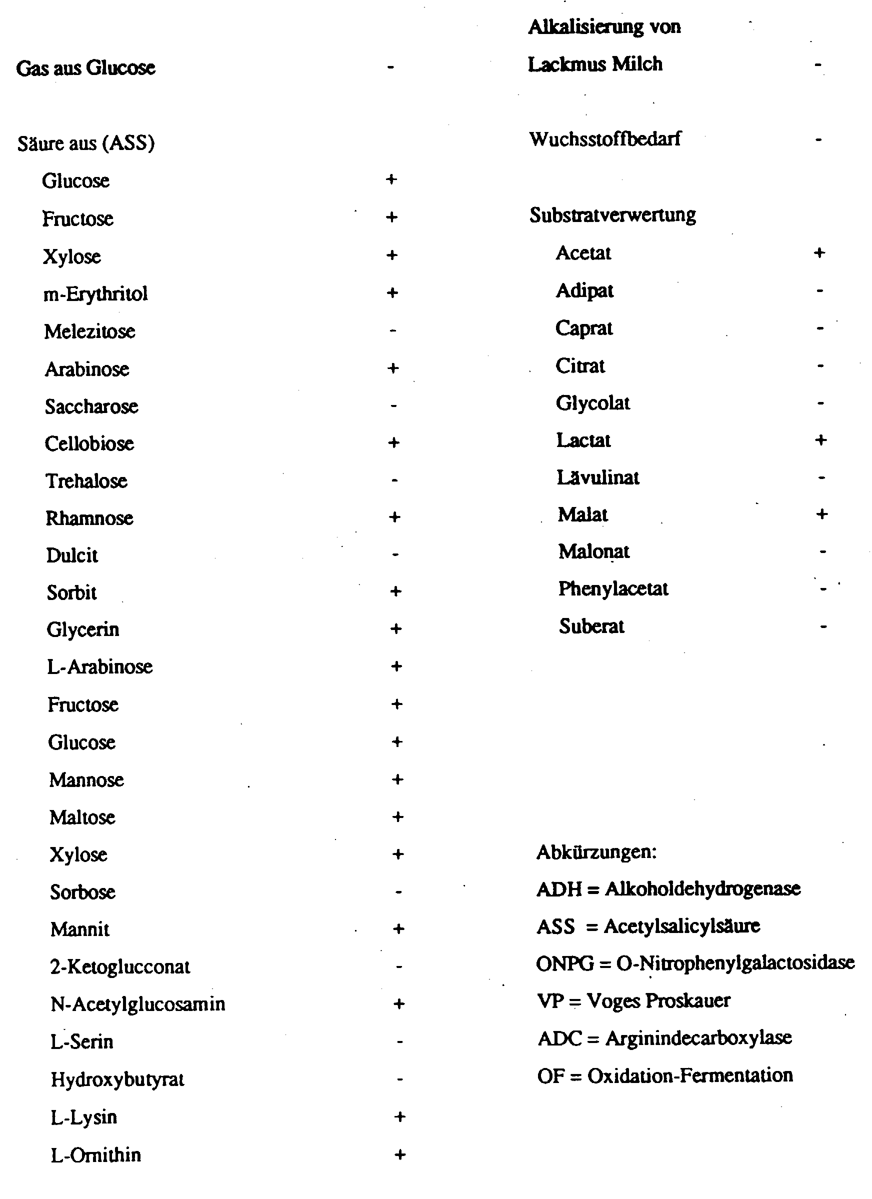

Üblicherweise werden die Mikroorganismen vor der eigentlichen Umsetzung angezüchtet (kultiviert) und deren wirksame Enzyme induziert.

Vorzugsweise erfolgt die Kultivierung (Anzucht) und die Induktion mit 3-Cyanpyridin als einzige Kohlenstoff-, Stickstoff- und Energiequelle.

Vor der Substratzugabe (2-Cyanpyrazin) können dann die Mikroorganismen entweder mittels üblichen Irennverfähren geerntet und in frischem Medium resuspendiert werden oder das Substrat (2-Cyanpyrazin) kann direkt zu den Mikroorganismen im ursprünglichen Wachstumsmedium zugegeben werden.
Für das eigentliche Verfahren wird dann zweckmässig die Zellsuspension auf eine optische Dichte bei 650 nm, von 1 bis 100 , vorzugsweise von 10 bis 40 eingestellt.

Als Medien können, sowohl für die Kultivierung als auch für die eigentliche Umsetzung die in der Fachwelt üblichen angewendet werden.

Das Substrat (2-Cyanpyrazin) kann einmalig oder kontinuierlich zugegeben werden.

Zweckmässig erfolgt die Substratzugabe so, dass die Substrat- konzentration 20 Gew%, vorzugsweise 8 Gew.% nicht übersteigt. Üblicherweise erfolgt die Umsetzung von 2-Cyanpyrazin zu 5-Hydroxypyrazin-2-carbonsäure mit ruhenden Zellen.

Zweckmässig wird die Umsetzung bei einem pH-Wert von 4 bis 10, vorzugsweise bei einem pH-Wert von 6 bis 8 durchgeführt.

Die Temperatur liegt zweckmässig zwischen 10 und 60°C, vorzugsweise zwischen 15 und 45°C.

Nach einer üblichen Umsetzungsdauer von 4 bis 100 h, kann das Produkt durch Ansäuern der zellfreien Lösung ausgefällt und mittels fachmännisch üblichen Aufarbeitungsmethoden erhalten werden.

### Beispiel:

Agrobacterium sp. DSM 6336 wurde in einem Mineralsalzmedium (Tabelle I) unter Zusatz von 0,1% (w/v) 3-Cyanpyridin bei einer Temperatur von 30°C in einem 300 ml Erlenmeyerkolben angezogen. Nach 36 h Wachstum wurden die Zellen abzentrifugiert und im gleichen Medium ohne 3-Cyanpyridin gewaschen. Dann wurden 25 ml der Zellsuspension mit einer optischen Dichte von 10 bei 650 nm mit 297 mg (2,83 mmol) 2-Cyanpyrazin versetzt und während 16 h auf einer Schüttelmaschine inkubiert. Nach dieser Inkubationszeit konnte im UV-Spektrum kein Ausgangsmaterial mehr nachgewiesen werden. Anschliessend wurden die Zellen abzentrifugiert und der Überstand am Rotationsverdampfer 5fach aufkonzentriert und mit konz. HCl auf pH 2,0 angesäuert. Die ausgefallene 5-Hydroxypyrazin-2-carbonsäure wurde abfiltriert und getrocknet. Insgesamt konnten 307 mg (2,21 mmol) Produkt isoliert werden, was einer Ausbeute von 78% entsprach. Im
¹H-NMR-Spektrum (D₂O) liessen sich keine Verunreinigungen erkennen.

**Tabelle I:**

| A+N-Medium | |
|---|---|
| Zusammensetzung: | Konzentration (mg/l) |
| (NH₄)₂SO₄ | 2000 |
| Na₂HPO₄ | 2000 |
| KH₂PO₄ | 1000 |
| NaCl | 3000 |
| MgCl₂·6H₂O | 400 |
| CaCl₂·2H₂O | 14,5 |
| FeCl₃·6H₂O | 0,8 |
| Pyridoxal-Hydrochlorid | 10·10⁻³ |
| Riboflavin | 5·10⁻³ |
| Nicotinsäureamid | 5·10⁻³ |
| Thiamin-Hydrochlorid | 2·10⁻³ |
| Biotin | 2·10⁻³ |
| Panthothensäure | 5·10⁻³ |
| p-Aminobenzoat | 5·10⁻³ |
| Folsäure | 2·10⁻³ |
| Vitamin B12 | 5·10⁻³ |
| ZnSO₄·7H₂o | 100·10⁻³ |
| MnCl₂·4H₂O | 90·10⁻³ |
| H₃BO₃ | 300·10⁻³ |
| CoCl₂·6H₂O | 200·10⁻³ |
| CuCl₂·2H₂O | 10·10⁻³ |
| NiCl₂·6H₂O | 20·10⁻³ |
| Na₂MoO₄·2H₂O | 30·10⁻³ |
| EDTANa₂·2H₂O | 5,0 |
| FeSO₄·7H₂O | 2,0 |
| (pH der Lösung wurde auf 7,0 eingestellt) | |

## Patentansprüche

1. Mikrobiologisches Verfahren zur Herstellung von 5-Hydroxypyrazin-2-carbonsäure und/oder deren Salze, dadurch gekennzeichnet, dass man 2-Cyanpyrazin, als Substrat mit Mikroorganismen, die mit 3-Cyanpyridin als einzige Kohlenstoff-, Stickstoff- und Energiequelle wachsen und dieses über das entsprechende 6-Hydroxypyridin Carbonsäure- Derivat abbanen, in 5-Hydroxypyrazin-2-carbonsäure und/oder deren Salze überführt, wobei letzteres im Medium akkumuliert wird.

2. Verfahren nach Patentanspruch 1, dadurch gekennzeichnet, dass man die Umsetzung mit Mikroorganismen der Spezies Agrobacterium DSM 6336 oder deren Deszendenten und Mutanten durchführt.

3. Verfahren nach mindestens einem der Patentansprüche 1 und 2, dadurch gekennzeichnet, dass die wirksamen Enzyme der Mikroorganismen mit 3-Cyanpyridin induziert werden.

4. Verfahren nach mindestens einem der Patenansprüche 1 bis 3, dadurch gekennzeichnet, dass die Umsetzung unter einmaliger oder kontinuierlicher Substratzugabe erfolgt, so dass die Substratkonzentration 20 Gew.% nicht übersteigt.

5. Verfahren nach mindestens einem der Patentansprüche 1 bis 4, dadurch gekennzeichnet, dass man die Umsetzung bei einem pH von 4 bis 10 und bei einer Temperatur von 10 bis 60°C durchführt.

## Claims

1. A microbiological process for the production of 5-hydroxypyrazine-2-carboxylic acid and/or the salts thereof, characterized in that the substrate 2-cyanopyrazine is converted with microorganisms which grow with 3-cyanopyridine as their sole carbon, nitrogen and energy source, and which decompose the same through the corresponding 6-hydroxypyridine carboxylic acid derivative, to 5-hydroxypyrazine-2-carboxylic acid and/or the salts thereof, wherein the latter accumulates in the medium.

2. The process of patent claim 1, characterized in that the conversion is carried out with microorganisms of the species Agrobacterium DSM 6336 or descendants or mutants thereof.

3. The process of at least one of the patent claims 1 and 2, characterized in that the effective enzymes of the microorganisms are induced with 3-cyanopyridine.

4. The process of at least one of the patent claims 1 to 3, characterized in that the conversion takes place with a single or continuous substrate addition ensuring that the substrate concentration does not exceed 20% by weight.

5. The process of at least one of the patent claims 1 to 4, characterized in that the conversion is performed at a pH of 4 to 10 and a temperature of 10 to 60 °C.

## Revendications

1. Procédé microbiologique pour la préparation d'acide 5-hydroxypyrazine-2-carboxylique et/ou de ses sels, caractérisé en ce que l'on transforme la 2-cyanpyrazine en tant que substrat avec des microorganismes qui sont mis en culture avec la 3-cyanpyridine en tant que source unique d'énergie d'azote et de carbone, et celui-ci est décomposé par le dérivé correspondant de l'acide 6-hydroxypyridine carboxylique, en acide 5-hydroxypyrazine-2-carboxylique et/ou de ses sels, ce dernier étant accumulé dans le milieu.

2. Procédé selon la revendication 1, caractérisé en ce que l'on procède à la réaction de microorganismes de l'espèce Agrobacterium DSM 6336 ou de ses descendants et mutants.

3. Procédé selon au moins l'une des revendications 1 et 2, caractérisé en ce que des enzymes efficaces des microorganismes sont induits par 3-cyanpyridine.

4. Procédé selon au moins l'une des revendications 1 à 3, caractérisé en ce que la réaction s'effectue par addition de substrat en une seule fois afin que la concentration de substrat ne dépasse pas 20% en poids.

5. Procédé selon au moins l'une des revendications 1 à 4, caractérisé en ce que l'on conduit la réaction à un .pH de 4 à 10 et à une température de 10 à 60°C.
